# EUROPEAN PATENT APPLICATION

(11) **EP 0 565 821 A2**
(43) Date of publication of application: **20.10.1993**
(21) Application number: 93102040.8
(22) Date of filing: 10.02.1993
(51) Int. Cl.: C07C 15/24, C07C 2/66

(54) **Method of preparing 2,6-diisopropylnaphthalene**

(30) Priority: 13.02.1992 JP 58744/92
(71) Applicant: IDEMITSU PETROCHEMICAL CO. LTD., Tokyo 100 (JP)
(72) Inventor: Iwahara, Masahiro, c/o Idemitsu Petrochemical, Tokuyama-shi, Yamaguchi-ken (JP); Aoki, Akinobu, c/o Idemitsu Petrochemical, Chiyoda-ku, Tokio (JP); Saruwatari, Tetsuya, c/o Idemitsu Petrochemical, Tokuyama-shi, Yamaguchi-ken (JP); Imada, Soichiro, c/o Idemitsu Petrochemical, Tokuyama-shi, Yamaguchi-ken (JP)
(74) Representative: Türk, Gille, Hrabal, Leifert

(57) **Abstract**

Disclosed is a method of producing 2,6-diisopropylnaphthalene by reacting propylene and naphthalene with each other in the presence of an acidic catalyst with recycling the side products after separation of the intended product of 2,6-diisopropylnaphthalene and the heavy residue from the reaction products by distillation, which is characterized in that the content of the following component (A) is controlled to be 3 % by weight or less and that of the following component (B) to be 12 % by weight or less:
Component (A): naphthalene derivatives having two isopropyl groups and one alkylene group or two alkyl groups in the naphthalene nucleus and having 22 carbon atoms and a molecular weight of 294
Component (B): naphthalene derivatives having one isopropyl group and one alkylene group or two alkyl groups in the naphthalene nucleus and having 19 carbon atoms and a molecular weight of 252.
By controlling the contents of the components (A) and (B), which lower the activity of the catalyst and lower the yield of the product, each to fall within the defined range, the yield of the product 2,6-diisopropylnaphthalene is elevated.

## Description

### FIELD OF THE INVENTION

The present invention relates to a method of preparing 2,6-diisopropylnaphthalene and, more precisely, to that which is characterized by controlling the amounts of particular compounds in the side product for recycling them.

### BACKGROUND OF THE INVENTION

2,6-Diisopropylnaphthalene is essentially used as a raw material for producing 2,6-dicarboxynaphthalane, which is considered to be important as a raw material monomer for engineering plastics. For producing 2,6-diisopropylnaphthalene, generally known is a method of alkylating naphthalene with propylene in the presence of an acidic catalyst such as aluminium chloride followed by elevating the reaction temperature for disproportionating and assymetrizing the alkylated product. Also known is a method of simultaneously effecting the alkylation, disproportionation and assymetrization.

In the method of preparing 2,6-diisopropylnaphthalene using an acidic catalyst, the reaction products are separated by distillation into a fraction comprising naphthalene and monoisopropylnaphthalene which are lighter than 2,6-diisopropylnaphthalene, a fraction comprising a mixture of diisopropylnaphthalene isomers containing 2,6-diisopropylnaphthalene, a heavy fraction consisting essentially of triisopropylnaphthalene and tetraisopropylnaphthalene which are heavier than 2,6-diisopropylnaphthalene, and a heavy residue. In this, the side products except the heavy residue and the intended product of 2,6-diisopropylnaphthalene are generally recycled to be used as the starting materials. On the other hand, the diisopropylnaphthalene fraction from which 2,6-diisopropylnaphthalene has been separated may not be recycled but is recovered to be used as a solvent for dyes of pressure-sensitive copying materials.

Anyhow, the method of producing 2,6-diisopropylnaphthalene with recycling the side products involves a drawback that the activity of the catalyst is noticeably lowered after lapse of a determined period of time to lower the yield of the product 2,6-diisopropylnaphthalene.

The present inventors have noted the heavy fraction to be recycled and have found that particular naphthalene derivatives lower the activity of the catalyst and lower the yield of the product. On the basis of the finding, they have completed the present invention.

### SUMMARY OF THE INVENTION

Specifically, there is provided in accordance with the present invention a method of producing 2,6-diisopropylnaphthalene by reacting propylene and naphthalene with each other in the presence of an acidic catalyst with recycling the side products after separation of the intended product of 2,6-diisopropylnaphthalene and the heavy residue from the reaction products by distillation, which is characterized in that the content of the following component (A) is controlled to be 3 % by weight or less and that of the following component (B) to be 12 % by weight or less:
Component (A): naphthalene derivatives having two isopropyl groups and one alkylene group or two alkyl groups in the naphthalene nucleus and having 22 carbon atoms and a molecular weight of 294
Component (B): naphthalene derivatives having one isopropyl group and one alkylene group or two alkyl groups in the naphthalene nucleus and having 19 carbon atoms and a molecular weight of 252.

### DETAILED DESCRIPTION OF THE INVENTION

The starting materials of the present invention are propylene and naphthalene and they are reacted with each other in the presence of an acidic catalyst to produce 2,6-diisopropylnaphthalene, whereupon the side products to be separated by removing the intended product of 2,6-diisopropylnaphthalene and the heavy residue from the reaction products by distillation are recycled.

The side products to be recycled include monoisopropylnaphthalene, diisopropylnaphthalene, triisopropylnaphthalene, tetraisopropylnaphthalene and other naphthalene derivatives. Generally, components which are heavier than tetraisopropylnaphthalene are not recycled. Where a mixture of other diisopropylnaphthalene isomers than 2,6-diisopropylnaphthalene are separated as a product, the compounds are not recycled naturally.

In carrying out the present invention, it is preferred that the proportion of the starting materials is so adjusted that the molar ratio of the isopropyl group to the naphthalene nucleus in the reaction products falls within a range of from 1.7 to 2.3. In general, it is adjusted so that the molar ratio may be 2.

For the adjustment, for example, one mole of propylene to be used as the starting material is calculated as one mole of isopropyl group; one mole of the recycled monoisopropylnaphthalene is one comprising one mole of isopropyl group and one mol of naphthalene nucleus; and one mole of the recycled triisopropylnaphthalene is one comprising three moles of isopropyl group and one mole of naphthalene nucleus. Based on the calculation, the respective components for the reaction may well be adjusted.

If the molar ratio is less than 1.7 or is more than 2.3, the yield of diisopropylnaphthalenes would lower so that the yield of the intended product 2,6-diisopropylnaphthalene would lower unfavorably.

Continuing the production of 2,6-diisopropylnaphthalene with recycling the side products, the low-reactive naphthalene derivatives of the reaction products gradually accumulate in the reaction system. The following component (A) having a molecular weight near to that of tetraisopropylnaphthalene and the following component (B) having a molecular weight near to that of triisopropylnaphthalene also accumulate in the reaction system, continuing the production of 2,6-diisopropylnaphthalene with recycling the side products.
Component (A): naphthalene derivatives having two isopropyl groups and one alkylene group or two alkyl groups in the naphthalene nucleus and having 22 carbon atoms and a molecular weight of 294.
Component (B): naphthalene derivatives having one isopropyl group and one alkylene group or two alkyl groups in the naphthalene nucleus and having 19 carbon atoms and a molecular weight of 252.

Since the preceding component (A) and component (B) are substances which lower the activity of the catalyst to lower the yield of the mixture of isomers of diisopropylnaphthalene containing the intended product of 2,6-diisopropylnaphthalene, it is necessary to control the amounts of the substances in the reaction system. In the present invention, the content of the component (A) is controlled to be 3 % by weight or less, preferably 2 % by weight or less, to the whole of the starting materials; and that of the component (B) is to be 12 % by weight or less, preferably 7 % by weight or less, to the whole of the starting materials.

Controlling the contents of the preceding component (A) and component (B) each to fall within the preceding range, the activity of the catalyst is not lowered so that production of 2,6-diisopropylnaphthalene of a stable yield is possible. Though not clear, the reason may be presumed because these component (A) and component (B), if present in the reaction system each in a certain concentration or more, would act as a catalyst poison to lower the activity of the catalyst.

Though also not clear, the mechanism of production of the component (A) and component (B) may be presumed to be such that the adjacent isopropyl groups as being on the naphthalene nucleus would cyclize or the cyclized ring would be opened to give them.

The acidic catalyst for use in the present invention may be any known one. For instance, mentioned are aluminium chloride and solid zeolite catalysts (such as Y-type zeolite, de-aluminiumed Y-type zeolite, iron-carried Y-type zeolite, H-type mordenite, etc.).

The condition of using the catalyst is not specifically defined. The preferred reaction condition may suitably be selected in consideration of the kind of the catalyst to be used. For instance, when aluminium chloride is used, the reaction is generally effected in two stages of alkylation and disproportionation-assymetrization. One-stage reaction where alkylation and disproportionation-assymetrization are effected at the same time is also possible. However, in consideration of the yield of 2,6-diisopropylnaphthalene, the former two-stage reaction is preferred.

The alkylation is effected in such a way that the starting material except propylene is first fed into a reactor along with a catalyst and propylene is added thereto. The propylene added immediately reacts with naphthalene so that the time for the alkylation is substantially same as the time of adding propylene to the reactor.

The alkylation is effected generally at 50 to 250°C for 10 minutes to 2 hours. If the temperature is lower than 50°C, the rate of producing diisopropylnaphthalenes would be slow inefficiently. On the other hand, if it is higher than 250°C, the amount of side products which are heavier than tetraisopropylnaphthalene would increase so that the yield of diisopropylnaphthalene would lower.

If the reaction time is shorter than 10 minutes, removal of the heat to be generated by the reaction would be difficult unfavorably. On the other hand, if it is longer than 2 hours, such would be advantageous for removal of the heat but the production efficiency would be poor because of the reaction of needing too much time.

The disproportionation-assymetrization is effected generally at 100 to 250°C for 30 minutes to 4 hours. If the reaction temperature is lower than 100°C, the rate of disproportionation and assymetrization would be slow inefficiently. On the other hand, if it is higher than 250°C, the amount of the heavy side products to be produced would increase so that the yield of diisopropylnaphthalene would lower unfavorably, like the case of the preceding alkylation.

The reaction pressure has almost no influence on the yield of the product, and therefore the reaction is generally effected at room temperature.

The reaction conditions in the case of using a solid zeolite catalyst such as Y-type zeolite will be mentioned below. In general, a fixed bed reactor is used. In the case, in general, 2,6-diisopropylnaphthalene is produced by one-stage reaction where alkylation and disproportionation-assymetrization are effected at the same time.

The other reaction conditions are as follows: The reaction temperature is from 200 to 300°C; and LHSV is suitably from 0.1 to 10. If the reaction temperature is lower than 200°C, the rate of producing diisopropylnaphthalene would slow; but if it is higher than 300°C, the catalyst would be coked to lower the activity of catalyst, unfavorably.

If LHSV is less than 0.1, the amount of diisopropylnaphthalene to be produced would be small unfavorably. On the other hand, if it is more than 10, the reaction could not progress well, also unfavorably.

The reaction pressure is suitably from 6 to 20 kg/cm²G, when a fixed bed reactor is used and the reaction is effected continuously.

Where aluminium chloride is used as the catalyst to produce 2,6-diisopropylnaphthalene by two-stage reaction, the reaction may be effected in the absence of a solvent. Where a solid zeolite catalyst is used to carry out the reaction in a fixed bed reactor, the reaction may be effected in the absence of a solvent but is preferably effected in the presence of a suitable solvent, if desired. In the preferred case, the solvent to be used may be selected from aliphatic saturated hydrocarbons such as n-undecane and aromatic solvents such as decalin.

As mentioned above, the side products are recycled in the method of the present invention to produce 2,6-diisopropylnaphthalene. The recycle is generally applied to the alkylation step. If desired, it may also be applied to the disproportionation-assymetrization step in the two-stage reaction. In the latter case, it is necessary that the amounts of the component (A) and component (B) to be fed to the disproportionation-assymetrization are controlled each to fall within the above-mentioned range in consideration of the component (A) and component (B) to be produced in the alkylation step.

Control of the contents of the component (A) and component (B) in the starting material may be effected by any desired means, which, in general, is effected by controlling the contents of the component (A) and component (B) in the heavy fraction of containing triisopropylnaphthalene having a higher boiling point than diisopropylnaphthalene.

The heavy fraction consisting essentially of triisopropylnaphthalene and tetraisopropylnaphthalene is recycled to the starting material, generally after separating the heavy residue having a higher boiling point than the fraction therefrom by distillation. In the case, since the boiling point of the component (A) is higher than any of triisopropylnaphthalene, the component (B) and tetraisopropylnaphthalene, the distillation condition is suitably controlled in such a way that the component (A) may be moved to the heavy residue whereby a heavy fraction having a small content of the component (A) may be obtained.

On the other hand, where the component (B) is recycled, a part of it becomes the component (A). Therefore, in general, the contents of the component (A) and component (B) in the starting material may well be controlled each to fall within the defined range by recycling the heavy fraction after the above-mentioned distillation.

If there is a possibility that the content of the component (B) is more than the defined range, the distillation is controlled in such a way that the component (B) and tetraisopropylnaphthalene may also remain in the heavy residue in consideration of the property of the component (B) of itself that it has a higher boiling point than triisopropylnaphthalene and has a lower boiling point than tetraisopropylnaphthalene, so that a heavy fraction consisting essentially of triisopropylnaphthalene is obtained and the fraction may be recycled. Accordingly, since the content of the component (B) in the side products decreases, the distillation condition for the heavy fraction is modified into the above-mentioned distillation condition of moving only the component (A) to the heavy residue, whereby production of 2,6-diisopropylnaphthalene may be continued.

The present invention will be explained in more detail by way of the following examples, which, however, are not intended to restrict the scope of the present invention.

### EXAMPLES 1 AND 2, AND COMPARATIVE EXAMPLES 1 AND 2

80.9 g of naphthalene and naphthalene derivatives were put in a one-liter flask, as starting materials. The naphthalene derivatives comprised 84.0 g of isopropylnaphthalene and 228.0 g of diisopropylnaphthalenes (containing 45.6 g of 2,6-diisopropylnaphthalene) and also triisopropylnaphthalene, tetraisopropylnaphthalene, the component (A) and the component (B) of the amounts described in Table 1 below. These naphthalene derivatives were isolated from a mixture of naphthalene derivatives with a precision distillator filled with Dixon packing.

3.0 g of aluminium chloride was added to the flask, as a catalyst. Then, the contents of the flask were heated up to 90°C under atmospheric pressure, and 45 g of propylene was added thereto over a period of 30 minutes for effecting alkylation.

Next, the temperature of the reaction system was elevated up to 180°C for effecting disproportionation and assymetrization for 2 hours.

The results of the reaction are shown in Table 1. The yield of 2,6-diisopropylnaphthalene shown therein is % by weight to the all starting materials.

**Table 1**

| | Example 1 | Example 2 | Comparative Example 1 | Comparative Example 2 |
|---|---|---|---|---|
| Tri IPN | 116.1 | 96.1 | 75.1 | 86.1 |
| Tet IPN | 27.0 | 27.0 | 27.0 | 3.0 |
| (A) | 3.0 (0.5) | 3.0 (0.5) | 3.0 (0.5) | 24.0 (4.0) |
| (B) | 16.0 (2.9) | 36.0 (6.0) | 74.0 (12.3) | 46.0 (7.7) |
| Yield of 2,6-DIPN | 21.5 | 20.7 | 16.7 | 15.4 |
| Tri IPN: Triisopropylnaphthalane Tet IPN: Tetraisopropylnaphthalene 2,6-DIPN: 2,6-Diisopropylnaphthalene The number as parenthesized in Table 1 indicates % by weight to the all starting materials. | | | | |

In accordance with the method of the present invention where propylene and naphthalene are reacted with each other in the presence of an acidic catalyst to produce 2,6-diisopropylnaphthalene with recycling the side products after separation of the intended product of 2,6-diisopropylnaphthalene and the heavy residue from the reaction products by distillation, the contents of particular compounds in the side products, which cause lowering of the activity of the acidic catalyst to lower the yield of the product, are controlled so that the product of 2,6-diisopropylnaphthalene is obtained at a good yield.

While the invention has been described in detail and with reference to specific embodiments thereof, it will be apparent to one skilled in the art that various changes and modifications can be made therein without departing from the spirit and scope thereof.

## Claims

1. A method of producing 2,6-diisopropylnaphthalene by reacting propylene and naphthalene with each other in the presence of an acidic catalyst with recycling the side products after separation of the intended product of 2,6-diisopropylnaphthalene and the heavy residue from the reaction products by distillation, which is characterized in that the content of the following component (A) is controlled to be 3 % by weight or less and that of the following component (B) to be 12 % by weight or less:
Component (A): naphthalene derivatives having two isopropyl groups and one alkylene group or two alkyl groups in the naphthalene nucleus and having 22 carbon atoms and a molecular weight of 294
Component (B): naphthalene derivatives having one isopropyl group and one alkylene group or two alkyl groups in the naphthalene nucleus and having 19 carbon atoms and a molecular weight of 252.

2. The method of producing 2,6-diisopropylnaphthalene as claimed in claim 1, in which the side products to be recycled are monoisopropylnaphthalene, diisopropylnaphthalene, triisopropylnaphthalene, tetraisopropylnaphthalene and other naphthalene derivatives except those which are heavier than tetraisopropylnaphthalene.

3. The method of producing 2,6-diisopropylnaphthalene as claimed in claim 1, in which the proportion of the starting materials is so adjusted that the molar ratio of the isopropyl group to the naphthalene nucleus in the reaction products falls within a range of from 1.7 to 2.3.

4. The method of producing 2,6-diisopropylnaphthalene as claimed in claim 1, in which the proportion of the starting materials is so adjusted that the molar ratio of the isopropyl group to the naphthalene nucleus in the reaction products is 2.
